# FASCICULE DE BREVET EUROPEEN

(11) **EP 2 291 173 B1**
(45) Date de publication et mention de la délivrance du brevet: **05.10.2016**
(21) Numéro de dépôt: 09757721.7
(22) Date de dépôt: 12.05.2009
(51) Int. Cl.: A61K 8/97, A61Q 19/08

(54) **ASSOCIATION D'EXTRAITS DE PASSIFLORE ET D'ANCHUSE UTILISABLE EN COSMETIQUE**
KOMBINATION VON PASSIONSBLUMEN- UND ALKANER-EXTRAKTEN ZUR VERWENDUNG IN KOSMETIKA
COMBINATION OF PASSION FLOWER AND ALKANET EXTRACTS FOR USE IN COSMETICS

(30) Priorité: 13.05.2008 FR 0802590
(43) Date de publication de la demande: 09.03.2011
(73) Titulaire: SOCIETE DE RECHERCHE COSMETIQUE SARL, 2314 Luxembourg (LU)
(72) Inventeur: LECLERE, Jacques, F-45500 Saint-Gondon (FR)
(74) Mandataire: L'Helgoualch, Jean
(86) Numéro de dépôt international: PCT/FR2009/050866
(87) Numéro de publication internationale: WO 2009/147345

(56) Documents cités:
- EP-A- 1 002 524
- EP-A- 1 537 789
- DE-A1- 4 201 749
- US-A1- 2006 018 867
- US-A1- 2007 134 189
- DATABASE WPI Week 200340 Thomson Scientific, London, GB; AN 2003-424016 XP002509676 -& JP 2002 332224 A (KOSE KK) 22 novembre 2002 (2002-11-22)
- "Natural Skin Care and Cosmetic Ingredients Glossary" , [Online] 1 juin 2006 (2006-06-01), pages 1-6, XP002509675 Extrait de l'Internet: URL:http://web.archive.org/web/20060106054 403/http:/www.herballuxuries.com/ingredien tslist.htm> [extrait le 2009-01-09]

## Description

La présente invention concerne une nouvelle composition cosmétique et/ou dermatologique pour la prévention et le traitement des signes du vieillissement cutané, et plus particulièrement une composition à base d'extraits de passiflore et d'anchuse, procurant un effet décontractant de la peau et capable d'agir efficacement contre les contractions musculaires, en particulier les contractions faciales et les rides d'expression.

La peau constitue à la fois une barrière anatomique vivante et une zone d'échange entre le corps et son environnement, dont l'efficacité conditionne le maintien d'un bon équilibre homéostasique. Elle comprend une couche superficielle constituée par l'épiderme, et des couches plus profondes formant le derme et l'hypoderme, et chacune de ces couches possède des propriétés spécifiques permettant à l'ensemble de réagir et de s'adapter aux conditions de son environnement.

La couche superficielle formant l'épiderme a une épaisseur variable selon les différentes parties du corps, et est principalement composée de kératinocytes (85 à 90% des cellules épidermiques), de mélanocytes (2 à 3% des cellules épidermiques) et des cellules de Langerhans. Le derme, plus épais, se compose principalement de collagène, d'élastine et de protéoglycanes. Ces trois types de molécules sont synthétisés par les fibroblastes dermiques. Les fibres de collagène assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme. La couche la plus profonde de la peau, l'hypoderme, contient les adipocytes qui produisent des lipides afin que le tissu sous-cutané puisse fabriquer une couche grasse protégeant les muscles, les os et les organes internes contre les chocs.

Le vieillissement de la peau peut être intrinsèque, ou extrinsèque c'est-à-dire provoqué par l'environnement, y compris les agressions climatiques, qui peuvent notamment contribuer à accélérer la dégradation du collagène du derme, et en particulier l'exposition au soleil, les variations de températures et les radicaux libres. Les premiers signes du vieillissement de la peau, tels que les rides et ridules, sont généralement provoqués par le stress et les changements biologiques et physiologiques, accélérés par l'environnement extérieur ou par les modes de vie. L'apparition de marques pigmentaires, la diminution de l'épaisseur de la peau et son affaissement sont également des changements observés au cours du vieillissement. On sait que la capacité de la peau à remplacer le collagène endommagé diminue avec le temps, et en conséquence des espaces et des irrégularités apparaissent dans le réseau du collagène.

Le vieillissement de la peau s'accompagne souvent de sensations de pesanteur, de pression durant le sommeil et aussi de contractions des muscles faciaux, déterminant des rides d'expression, notamment autour des yeux et de la bouche, ou la modification de microrelief épidermique, notamment en raison du stress. En particulier, les rides d'expression sont provoquées par de multiples contractions et crispations que subissent quotidiennement les traits du visage sous l'action des muscles sous-cutanés du visage qui se contractent et se relâchent successivement de manière incessante suivant le rythme des émotions.

Pour lutter contre ces signes du vieillissement, il est donc nécessaire de diminuer les forces de contraction des muscles faciaux en exerçant une inhibition de la libération des neuromédiateurs. Ce phénomène d'inhibition a pour conséquence de provoquer une décrispation des traits du visage et de réduire la profondeur des rides faciales.

Certains traitements connus peuvent avoir pour effet de limiter ou d'inhiber les contractions musculaires faciales. Ainsi, on sait qu'il est possible d'effectuer un traitement esthétique par injection de toxine botulique au niveau des rides afin de figer les cellules en contact avec la toxine, donnant à la peau un aspect plus lisse. On a aussi proposé des implants de collagène et d'acide hyaluronique pour dissimuler les lignes d'expression autour des yeux ou de la bouche, la dermabrasion pour éliminer la couche supérieure de la peau endommagée, la chirurgie esthétique, telle que la blépharoplastie (chirurgie des paupières), ou un lifting pour retendre une peau présentant un affaissement, ou encore une restructuration à l'aide d'un laser au dioxyde de carbone pour éliminer les ridules.

On a aussi proposé d'utiliser des compositions cosmétiques contenant des substances à action exfoliante comme les alpha-hydroxy acides, par exemple l'acide lactique et l'acide glycolique, pour faciliter l'élimination des cellules mortes et stimuler la formation de collagène et d'élastine. On connaît aussi des compositions à base d'acide rétinoïque ou de rétinol qui peuvent agir contre les signes du vieillissement cutané tels que la sécheresse, la perte d'élasticité de la peau et la formation de rides, ainsi que des crèmes décontractantes à base de magnésium ou de manganèse inhibant le flux de calcium dans les cellules, à l'origine de la contraction musculaire, afin de réduire les microcrispations de la peau et de ralentir l'apparition des rides d'expression.

Cependant, malgré les diverses compositions disponibles, il existe toujours un besoin de pouvoir disposer de nouvelles compositions topiques alternatives permettant de lutter efficacement contre les effets du vieillissement cutané et les rides d'expression, et notamment des compositions topiques à base d'extraits végétaux appropriés provenant préférentiellement de plantes connues pour leurs propriétés favorables.

La passiflore, de la famille des passifloraceae, est une plante médicinale vivace utilisée en infusions pour son effet calmant et sédatif. Elle comprend plusieurs centaines d'espèces, dont certaines, telles que *Passiflora edulis* et *Passiflora ligularis,* sont connues pour donner des fruits comestibles (fruit de la passion et grenadelle). Le brevet EP 1 541 037 décrit des compositions pour administration par voie orale, contenant un extrait de passiflore et des acides gras, pouvant être utilisées comme additifs alimentaires. La demande de brevet EP 1 537 789 décrit une composition à activité anti-inflammatoire pour application topique ou pour administration par voie orale contenant un caroténoïde, du tocophérol, un extrait de passiflore et un extrait de myrtille ; de telles compositions sont efficaces pour lutter contre les effets préjudiciables engendrés par une exposition excessive aux radiations solaires. D'autres additifs diététiques à base d'extraits de racines de kava en association avec des extraits végétaux, notamment de fleurs de passiflore et de camomille sont décrits dans le brevet US 5,770,207. Des compositions cosmétiques destinées à lutter contre le vieillissement de la peau et contenant des huiles riches en acides gras essentiels ainsi qu'un extrait de passiflore sont décrites dans le brevet EP 1 002 524. La demande de brevet US 2007/134189 décrit des compositions anti-rides à base d'extraits de passiflore, de menthe, de coquelicot et de myrte. La demande de brevet JP 2002-332224 décrit une composition cosmétique pour lutter contre le vieillissement cutané contenant un extrait de passiflore à titre de composé permettant d'éliminer les ions superoxyde et un additif choisi parmi les agents hydratants, les anti-oxydants, les activateurs de cellules, les agents de blanchiment et les filtres solaires.

Des extraits d'anchuse (*Anchusa officinalis*) ont été proposés dans des compositions alimentaires, en association avec d'autres extraits végétaux tels que *Magnolia officinalis, Nelumbo nucifera* et *Origanum vulgare,* pour inhiber la formation de mercaptans sous l'action de bactéries, comme indiqué dans le brevet JP 2005-162697. Des études ont montré que des extraits de racines d'*Anchusa strigosa* peuvent avoir un effet protecteur de l'ulcère induit par l'éthanol chez le rat (AM Disi et al., J. Ethnopharmacol. 1998, p.189-98). L'utilisation d'extraits d'anchuse à titre d'agent hydratant ou adoucissant dans des compositions cosmétiques a déjà été proposée, notamment dans la demande de brevet US 2006/018867.

Les travaux réalisés par la demanderesse ont montré qu'il est possible d'agir efficacement contre les contractions musculaires faciales responsables des signes du vieillissement cutané et en particulier des rides d'expression, au moyen de compositions topiques à base d'extraits de passiflore et d'anchuse.

La présente invention a donc pour objet une nouvelle composition cosmétique et/ou dermatologique à base de plantes, et plus particulièrement à base d'extraits de passiflore et d'anchuse présentant des effets d'inhibition des contractions musculaires faciales incontrôlées à l'origine des rides d'expression.

La présente invention a aussi pour objet l'utilisation d'un extrait de passiflore associé à un extrait d'anchuse pour la préparation d'une composition topique cosmétique et/ou dermatologique destinée à lutter contre les signes du vieillissement cutané, et en particulier les rides d'expression provoquées par les contractions musculaires faciales incontrôlées.

L'invention a encore pour objet un procédé cosmétique de traitement de la peau pour lutter contre les signes du vieillissement cutané, et en particulier les rides d'expression provoquées par les contractions musculaires faciales incontrôlées, consistant à appliquer sur les zones de la peau nécessitant un tel traitement, une composition cosmétique topique comprenant, une quantité efficace de l'association d'un extrait de passiflore et d'un extrait d'anchuse selon l'invention, en combinaison le cas échéant avec des supports et excipients cosmétiquement acceptables.

Les compositions suivant la présente invention comprennent en association un extrait de passiflore et un extrait d'anchuse, en combinaison le cas échéant avec les supports et excipients usuels dans les compositions cosmétiques et dermatologiques, pharmaceutiquement et cosmétiquement acceptables.

Le rapport en poids de l'extrait de passiflore à l'extrait d'anchuse dans les compositions de l'invention peut varier de 1:30 à 6:1 environ, et de préférence de 1:1 à 4:1.

La concentration de l'extrait de passiflore dans la composition totale de l'invention peut varier de 0,1% à 3%, de préférence de 0,2% à 2%, tandis que la concentration en extrait d'anchuse varie généralement de 0,2% à 3%, de préférence de 0,5 à 1%. Ces concentrations sont exprimées en extrait hydroglycériné de passiflore et d'anchuse tel que décrit ci-après. A titre d'exemple, dans le cas d'une association de passiflore et d'anchuse dans un rapport en poids 2:1, la concentration de l'association dans la composition totale peut varier de 0,1 à 20% en poids, et de préférence de 2 à 10%.

Suivant l'invention, on utilise les parties aériennes, en particulier les sommités fleuries d'anchuse et de passiflore, comprenant les fleurs et jeunes feuilles de la partie apicale, de préférence aux autres parties des plantes, pour préparer les extraits utilisables dans les compositions cosmétiques et dermatologiques.

Les extraits sont préparés à partir de plantes séchées, l'extrait représentant environ 5% du poids total de la plante. On peut par exemple préparer des extraits hydro-alcooliques, par exemples des extraits hydroglycoliques ou hydroéthanoliques, ou des extraits hydroglycérinés comme indiqué ci-après. Après séchage et pulvérisation des plantes, l'extraction se fait de préférence par percolation à raison de 100 g de plante pour 500 g d'eau. Le gâteau est exprimé et les liqueurs réunies et complétées à 500 g par addition de glycérine biologique.

L'extrait de passiflore utilisé dans l'invention présente les caractéristiques suivantes. Dans cet exemple, l'extrait utilisé est un extrait hydroglycériné obtenu à partir de l'espèce *Passiflora incarnata.*

| | |
|---|---|
| *Passiflora incarnata* (extrait sec) (parties aériennes fleuries) | 0,5 % |
| Eau | 49,75 % |
| Glycérine | 49,75 % |
| Densité à 22°C (1,050 - 1,150) | 1,117 |
| Indice de réfraction à 22°C (1,380 - 1,420) | 1,400 |
| pH (4,9 à 6,9) | 5,89 |

Les caractéristiques de l'extrait d'anchuse de l'invention sont indiquées ci-après. L'espèce d'anchuse utilisé dans cet exemple est *Anchusa arvensis.*

| | |
|---|---|
| *Anchusa arvensis* (extrait sec) (sommités fleuries) | 1,0 % |
| Eau | 49,5 % |
| Glycérine | 49,5 % |
| Densité à 22°C (1,050 - 1,150) | 1,122 |
| Indice de réfraction à 22°C (1,380 - 1,420) | 1,401 |
| pH (6,0 à 8,0) | 7,0 |

Les extraits hydroglycérinés utilisés dans l'invention présentent l'avantage de pouvoir être conservés pendant une période prolongée dans des conditions normales de température et d'humidité sans qu'il soit nécessaire d'ajouter un conservateur. Il est cependant possible de diminuer la quantité de glycérine et d'ajouter un conservateur usuel. Ainsi, on peut utiliser un extrait à 20 ou 30% de glycérine additionné d'un conservateur approprié tel que le benzoate de sodium ou le sorbate de potassium.

Les compositions de l'invention peuvent être utilisées avantageusement en cosmétologie et en dermatologie pour le traitement ou la prévention des signes du vieillissement cutané, plus particulièrement pour prévenir ou réduire les rides d'expression résultant des contractions musculaires faciales incontrôlées.

Les diverses espèces et variétés connues de passiflore ont des propriétés sédatives, tandis que l'anchuse (ou buglosse ou alkana), qui appartient à la famille des borraginacées, notamment *Anchusa sylvestris,* est connue pour ses propriétés colorantes, diurétiques et antitussives. Les passiflores sont des plantes que l'on trouve en abondance dans diverses régions à climat doux et dont la culture est relativement aisée. L'anchuse est une plante vivace à tige dressée pouvant atteindre 40 à 60 cm donnant des fleurs bleues à pourpres en grappes que l'on trouve couramment dans diverses régions européennes, en particulier sur le pourtour méditerranéen ainsi qu'en Asie.

Suivant l'invention on utilise un extrait de l'espèce *Passiflora incarnata.*

Suivant l'invention, on utilise un extrait de l'espèce *Anchusa arvensis.*

On a constaté que, de manière étonnante, l'association d'extraits de passiflore et d'extraits d'anchuse présente des propriétés utiles en cosmétologie et en dermatologie pour lutter efficacement contre les contractions faciales et les rides d'expression.

Les propriétés de l'association de passiflore et d'anchuse selon la présente invention ont été mises en évidence par des tests d'évaluation de l'effet de la composition vis-à-vis de la sensibilité et de la relaxation cutanée induites par les neuropeptides (substance P, CGRP et acétylcholine).

Les résultats des tests détaillés ci-après, ont fait apparaître une excellente activité de l'association de passiflore et d'anchuse sur le CGRP (« Calcitonine Gene Related Protein »), la substance P et l'acétylcholine. Ainsi, selon les concentrations utilisées on a pu constater :
- une diminution jusqu'à 25% de la libération de la substance P,
- une diminution jusqu'à 23% de la libération de la CGRP (Calcitonine Gene Related Protein),
- une diminution jusqu'à 28% de la fixation de la substance P sur son récepteur au niveau des kératinocytes humaines en culture,
- une diminution jusqu'à 27% de la libération d'acétylcholine par rapport au témoin non traité.

Ces résultats ont été obtenus avec une association d'extraits hydroglycérinés de passiflore et d'anchuse sur des cultures cellulaires, et confirment que, en application topique, la composition de l'invention présente un effet de relaxation musculaire.

Comme indiqué ci-dessus, l'invention concerne aussi un procédé cosmétique de traitement de la peau affectée par des rides d'expression résultant de contractions musculaires faciales incontrôlées. Suivant l'invention, on peut appliquer la composition décrite ci-dessus à raison d'une ou deux applications par jour pendant une période de temps adaptée à la situation de la personne.

Suivant une forme avantageuse de réalisation, la composition à base d'extraits de passiflore et d'anchuse peut être complétée par des principes actifs ou ingrédients auxiliaires choisis pour leurs propriétés complémentaires, afin de renforcer l'effet d'inhibition des contractions musculaires faciales ou de compléter les effets anti-âge de la composition.

Ainsi il est particulièrement avantageux de la combiner avec des quantités appropriées d'extraits de fleurs de lotus bleu (*Nymphaea caerulea*) et de guimauve (*Althea officinalis*), et éventuellement d'extrait de coquelicot (*Papaver rhoeas*), ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine).

On peut ajouter aussi des globulines de pois ou des mucilages tels que des mucilages de fruits de baobab afin d'obtenir un effet tenseur de la peau, d'huile de *Calophylum* afin de renforcer l'effet anti-rides, d'*Imperata cylindrica,* afin de favoriser l'hydratation de la peau par modification de la pression osmotique, du mucilage de coquelicot, des extraits de *Senna alata* favorisant la protection solaire, ainsi que d'une manière générale toute association avec un céramide.

Les compositions conformes à la présente invention peuvent être présentées sous les formes classiquement utilisées pour une application topique, c'est-à-dire sous forme de gel, lotion, émulsion (en particulier crème ou lait), sérum (ou essence), masque ou pommade, contenant des excipients et supports usuels compatibles et pharmaceutiquement acceptables. Elles peuvent aussi se présenter sous forme de lingettes imbibées d'une solution contenant les extraits suivant l'invention.

Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on utilise de préférence des émulsions à structure lamellaire contenant peu ou pas de produits éthoxylés.

Les compositions topiques selon l'invention peuvent comprendre divers excipients usuels adaptés à une administration topique externe, en particulier des excipients acceptables sur le plan dermatologique et cosmétologique. Ces excipients appropriés pour la formulation sont bien connus de l'homme du métier et comprennent par exemple des agents favorisant la pénétration tels que l'éthoxydiphénol, le phytantriol, l'octyl dodécanol et l'escine ; des agents hydratants tels que le propylène glycol, la glycérine, le butylène glycol ; des épaississants tels que les gommes naturelles et les polymères de synthèse ; des émollients et des tensioactifs tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogénée, l'alcool cétylique, le palmitate cétylique, le phosphate cétylique ; des émulsifiants ; des colorants ; des parfums ; etc. On peut aussi ajouter des vitamines antioxydantes telles que la vitamine E, par exemple l'acétate de tocophérol ou le tocotriénol, la vitamine C, les polyphénols naturels, ou encore des agents de gommage de la peau tels que le nylon et le nitrure de bore.

Le cas échéant, on peut ajouter des conservateurs tels que le benzoate de sodium, le sorbate de potassium, le phénoxyéthanol, le Phenonip® associant du phénoxyéthanol et des parahydroxybenzoates de méthyle, éthyle, butyle et isobutyle.

Les exemples suivants illustrent plus en détail l'invention sans en limiter la portée. Dans tous les exemples de compositions qui suivent, les parties sont exprimées en poids, sauf indication contraire.

### Exemple 1

L'étude des effets de l'association d'extraits de passiflore et d'anchuse de la présente invention a été faite sur des ganglions de la racine dorsale de rats suivant le protocole expérimental ci-dessous.

### Protocole expérimental

Les cellules de ganglions ont été prélevées le plus stérilement possible, et ont été conservées dans un tampon sous antibiotique. Elles ont été cultivées pendant 17 jours dans du milieu DMEM contenant du sérum de veau foetal. Pendant cette durée de culture, les cellules ont émis des prolongements de dendrites qui sécrètent les neuropeptides.

Après 17 jours de culture, le milieu a été éliminé et du milieu frais a été ajouté, soit seul, soit additionné de différentes concentrations de l'association d'extraits suivant l'invention. Les boîtes ont été mises à incuber pendant 24 heures à 37°C dans une étuve à 95% d'oxygène et 5% de CO2.

A la fin de la période d'incubation (24 heures), le milieu de culture a été récupéré. Les neuropeptides (substance P, CGRP, adrénaline et acétylcholine) ont été dosés grâce à un anticorps monoclonal spécifique.

### Dosage de la substance P

Les cellules sont incubées en présence ou en l'absence du produit à l'étude. A la fin du temps d'incubation, le milieu est prélevé, puis la substance P est dosée par réaction immunologique avec des anticorps monoclonaux spécifiques à ce neuropeptide.

L'essai est conduit en triplicate après 24 heures de traitement.
- lot 1 : témoin négatif
- lot 2 : témoin positif (induction par la Capsaïcine)
- lots 3 - 5 : traités par l'association d'extraits de passiflore et d'anchuse à 3 concentrations (0,1%, 0,5% et 1%).

Le rapport en poids passiflore / anchuse est de 2:1 pour les trois concentrations testées.
Les résultats sont regroupés dans le tableau ci-dessous :

| | Substance P (pg/ml) | Pourcentage |
|---|---|---|
| Témoin | 45,9 ± 3,4 | - |
| Témoin positif Capsaïcine (25µM) | 67,4 ± 4,2 | +47 |
| Passiflore + anchuse (0,1 %) | 38,2 ± 3,0* | -14 |
| Passiflore + anchuse (0,5%) | 36,3 ± 2,2* | -21 |
| Passiflore + anchuse (1%) | 34,2 ± 3,7* | -25 |

| | | |
|---|---|---|
| * Significativement différent par rapport au témoin p≤0,05 (Wilcoxon Rank Sum Test). | | |

### Dosage de la CGRP

Les cultures primaires de cellules nerveuses sont obtenues de la même façon que pour la substance P et le dosage de la CGRP est fait par réaction immunologique avec des anticorps monoclonaux spécifiques de la CGRP

L'essai est conduit en triplicate après 24 heures de traitement.
- lot 1 : témoin négatif
- lot 2 : témoin positif (induction par la Capsaïcine)
- lots 3 - 5 : traités par l'association d'extraits de passiflore et d'anchuse à 3 concentrations (0,1%, 0,5% et 1%).

Comme pour le dosage précédent, le rapport en poids passiflore / anchuse est de 2:1 pour les trois concentrations testées.

Les résultats sont regroupés dans le tableau ci-dessous :

| | CGRP (pg/ml) | Pourcentage |
|---|---|---|
| Témoin | 275,4 ± 13,8 | - |
| Témoin positif Capsaïcine (25µM) | 321,3 ± 10,7* | +17 |
| Passiflore + anchuse (0,1 %) | 238,3 ± 17,3* | -13 |
| Passiflore + anchuse (0,5%) | 233,0 ± 21,2* | -19 |
| Passiflore + anchuse (1%) | 212,3 ± 19,6* | -23 |

| | | |
|---|---|---|
| * Significativement différent par rapport au témoin p≤0,05 (Wilcoxon Rank Sum Test). | | |

### Dosage de l'acétylcholine

Les cellules nerveuses sont ensemencées comme précédemment. Les cellules sont incubées en présence ou en l'absence de l'association d'extraits l'invention. A la fin du temps d'incubation, le milieu est prélevé, puis l'acétylcholine est dosée par réaction immunologique avec des anticorps monoclonaux spécifiques.

L'essai est conduit en triplicate après 24 heures de traitement.
- lot 1 : témoin négatif
- lots 2 - 4 : traités par l'association d'extraits de passiflore et d'anchuse à 3 concentrations (0,1%, 0,5% et 1%).

Le dosage de l'acétylcholine est déterminé à l'aide du kit Amplex® Red (Acétylcholine/Acétylcholinestérase) qui fournit une méthode ultrasensible. Les utilisations potentielles de ce kit incluent le criblage des inhibiteurs d'acétylcholine estérase et la mesure de la libération de l'acétylcholine des synaptosomes.

Le dosage de l'acétylcholine est évalué à l'aide d'un lecteur de microplaque en utilisant une excitation à 560 nm et une détection de la fluorescence à 590 nm.

Les résultats sont regroupés dans le tableau ci-dessous :

| | Acétylcholine (µM) | Pourcentage |
|---|---|---|
| Témoin | 27,2 ± 2,2 | - |
| Passiflore + anchuse (0,1 %) | 23,0 ± 1,8* | -15 |
| Passiflore + anchuse (0,5%) | 21,1 ± 2,4* | -22 |
| Passiflore + anchuse (1%) | 19,8 ± 1,5* | -37 |

| | | |
|---|---|---|
| * Significativement différent par rapport au témoin p≤0,05 (Wilcoxon Rank Sum Test). | | |

### Evaluation de l'affinité vis-à-vis du récepteur de la substance P au niveau des kératinocytes humains en culture

Les kératinocytes sont incubés en présence ou en l'absence du produit à l'étude ou de ligand non radioactif (Sar9, Met(O2)11)-SP, avec la 3H (Sar9, Met(O2)11)-SP.

Après incubation, la radioactivité est dosée pour déterminer le potentiel du produit à l'étude à rentrer en compétition avec la substance P.

L'essai est conduit en triplicate après 24 heures de traitement.
- lot 1 : témoin négatif
- lot 2 : témoin positif (compétition du ligand marqué et non marqué)
- lots 3 - 5 : traités par l'association d'extraits de passiflore et d'anchuse à 3 concentrations (0,1%, 0,5% et 1%).

Comme pour les dosages précédents, le rapport en poids passiflore / anchuse est de 2:1 pour les trois concentrations testées.

Cette étude est basée sur la compétition de l'association d'extraits de passiflore et d'anchuse avec la substance P marquée. Ce résultat se traduit par une diminution de la fixation spécifique de la substance P marquée sur son récepteur quand le produit rentre en compétition.

Les résultats sont regroupés dans le tableau ci-dessous :

| | Cpm | Pourcentage |
|---|---|---|
| Témoin | 1823 ± 117 | - |
| Passiflore + anchuse (0,1%) | 1539 ± 132* | -16 |
| Passiflore + anchuse (0,5%) | 1424 ± 163* | -22 |
| Passiflore + anchuse (1%) | 1305 ± 112* | -28 |

| | | |
|---|---|---|
| * Significativement différent par rapport au témoin p≤0,01 (Wilcoxon Rank Sum Test). | | |

Ainsi, les résultats exposés dans les tests ci-dessus, montrent que le produit testé, par rapport au produit non traité :
- inhibe jusqu'à 25% de la libération de la substance P,
- inhibe jusqu'à 23% de la libération de la CGRP,
- inhibe jusqu'à 28% de la fixation de la substance P sur son récepteur au niveau des kératinocytes humaines en culture,
- inhibe jusqu'à 27% de la libération d'acétylcholine par rapport au témoin non traité.

Ces résultats montrent que l'association passiflore / anchuse suivant l'invention peut éliminer l'activité de la substance P et la CGRP, en inhibant leur libération et en entrant en compétition avec la substance P sur son récepteur. Cette activité au niveau des neuropeptides et plus précisément l'inhibition de leur libération peut être due à l'inhibition de l'Acétylcholine. En effet il a été montré que l'acétylcholine peut activer les fibres C nociceptives avec, pour conséquence, une augmentation de la concentration du CGRP et de la substance P.

### Exemple 2

Suivant les techniques classiques, on prépare une émulsion fluide décrispante des muscles faciaux ayant la composition pondérale suivante.

| Phase A | |
|---|---|
| Arachidyl glucoside | 1,5 |
| Cétéaryl glucoside | 1,0 |
| Alcool béhénylique | 2,0 |
| Alcool arachidylique | 0,5 |
| Beurre de Cupuaçu | 1,0 |
| Beurre de Karité | 1,0 |
| Huile de noyau de prune | 1,0 |
| Triglycérides caprique/caprylique | 2,0 |
| Tocophérol | 0,5 |

| Phase B | |
|---|---|
| Eau déminéralisée | q.s.p. 100,0 |
| Acide phytique | 0,2 |
| Acide déhydroacétique | 0,1 |
| Benzoate de sodium | 0,2 |
| Gomme xanthane | 0,1 |

| Phase C | |
|---|---|
| Extraits passiflore/anchuse (2:1) | 5,0 |
| Eau de laitue | 20,0 |
| Extrait de *Bacopa monnieri* | 1,0 |
| Argireline (acétyl hexapeptide-3) | 0,5 |

Les composants de la phase grasse sont soigneusement mélangés à 70°C, et on ajoute la phase aqueuse B, puis la phase C mélangée à 40°C et on maintient sous agitation jusqu'à obtenir une phase homogène.

L'extrait passiflore/anchuse utilisé dans la composition ci-dessus est un extrait hydroglycériné de Passiflora incarnata et d'Anchusa arvensis.

Cette crème fluide peut être appliquée sur le visage et provoque un effet de relaxation des muscles faciaux observé dès la première application.

### Exemple 3

On prépare une crème riche à base d'extraits passiflore/anchuse (2:1) ayant la composition pondérale ci-dessous par les techniques usuelles.

| Phase A | |
|---|---|
| Lécithine hydrogénée + alcool C10-C20 | |
| (Biophilic H®) | 4,0 |
| Huile de bourrache | 2,0 |
| Huile d'Inca-inchi | 2,0 |
| Huile de Tamanol (*Calophyllum inophyllum*) | 0,5 |
| Macérât huileux de carottes | 0,5 |
| Macérât huileux d'hydrocotyle asiatique | |
| (*Centella asiatica*) | 0,5 |
| Beurre de Karité | 3,0 |
| Alcool béhénylique | 2,0 |
| Stéarate de glycéryle | 1,5 |

| Phase B | |
|---|---|
| Eau déminéralisée | q.s.p. 100,0 |
| Acide déhydroacétique | 0,1 |
| Acide lévulinique | 0,1 |
| Lévulinate de sodium | 0,2 |
| Benzoate de sodium | 0,1 |
| Acide phytique | 0,1 |
| Gomme xanthane | 0,1 |

| Phase C | |
|---|---|
| Extraits passiflore/anchuse (2:1) | 7,0 |
| Extrait de nénuphar | 1,0 |
| Peptides de graines de pois | 2,0 |
| Eau d'Hamamélis | 10,0 |
| Phase D | |
| Essence de bois de rose | 0,1 |

La phase aqueuse B est homogénéisée à 73°C et versée dans la phase huileuse A préalablement mélangée à 75°C, puis on ajoute sous agitation la phase C contenant les extraits de passiflore et d'anchuse à 40°C, et on complète par l'essence de bois de rose.

### Exemple 4

On prépare une essence à base d'extraits passiflore/anchuse (2:1) ayant la composition pondérale ci-dessous par les techniques usuelles. Cette essence est destinée à un traitement intensif ("traitement de choc").

| Phase A | |
|---|---|
| Eau de roses | 30 |
| Eau d'Hamamélis | 10,0 |
| Eau de lierre terrestre | 10,0 |
| Eau de laitue | 5,0 |
| Extraits passiflore/anchuse (2:1) | 10,0 |
| Extrait de fleur de lotus bleu | 1,0 |
| Extrait de racine de guimauve | 0,2 |
| Extrait de *Goupia glabra* | 0,2 |
| Argireline (acétyl hexapeptide-3) | 3,0 |

| Phase B | |
|---|---|
| Gomme sclerotium | 0,3 |

| Phase C | |
|---|---|
| Acide déhydroacétique | 0,1 |
| Alcool benzylique | 0,9 |
| Acide phytique | 0,1 |

| Phase D | |
|---|---|
| Sérine | 0,02 |
| Lysine | 0,02 |
| Glycocolle | 0,02 |

| Phase E | |
|---|---|
| Glycérine | 5,0 |
| Eau déminéralisée | q.s.p. 100,0 |

Les composants de la phase A sont soigneusement mélangés à température ambiante, on y dissout la phase B, puis on ajoute la phase C, et dans le mélange obtenu on dissout la phase D, puis on ajoute la phase E et on complète par l'eau. On obtient ainsi une essence pour traitement d'attaque destinée à être appliquée sur la peau pour provoquer une décrispation rapide des muscles faciaux.

## Revendications

1. Composition cosmétique et/ou dermatologique comprenant en association un extrait hydroglycériné de *Passiflora incarnata* et un extrait hydroglycériné d'*Anchusa arvensis,* lesdits extraits étant obtenus par percolation à partir des parties aériennes des plantes, et en combinaison le cas échant avec des supports et excipients pharmaceutiquement et cosmétiquement acceptables.

2. Composition selon la revendication 1, **caractérisée en ce que** le rapport en poids de l'extrait hydroglycériné de *Passiflora incarnata* à l'extrait hydroglycériné *d'Anchusa arvensis* varie de 1 :30 à 6 : :1.

3. Composition selon la revendication 2, **caractérisée en ce que** le rapport en poids de l'extrait hydroglycériné de *Passiflora incarnata* à l'extrait hydroglycériné *d'Anchusa arvensis* varie de 1 :1 à 4 :1.

4. Composition selon la revendication 1, **caractérisée en ce que** la concentration de l'extrait hydroglycériné de *Passiflora incarnata* dans la composition totale varie de 0.1% à 3% en poids.

5. Composition selon la revendication 1, **caractérisée en ce que** la concentration de l'extrait hydroglycériné *d'Anchusa arvensis* dans la composition totale varie de 0.2% à 3% en poids.

6. Composition selon la revendication 1, **caractérisée en ce qu'**elle comprend une association d'extraits hydroglycérinés de *Passiflora incarnata* et *d'Anchusa arvensis* dont la concentration varie de 0.1 à 20% en poids d'extrait sec par rapport au poids total de la composition.

7. Utilisation d'un extrait hydroglycériné de *Passiflora incarnata* associé à un extrait hydroglycériné *d'Anchusa arvensis,* lesdits extraits étant obtenus par percolation, à partir des parties aériennes des plantes pour la préparation d'une composition cosmétique et/ou dermatologique destinée à lutter contre les signes du vieillissement cutané.

8. Utilisation selon la revendication 7, **caractérisée en ce que** la composition est destinée à lutter contre les rides d'expression provoquées par les contractions musculaires faciales incontrôlées.

9. Procédé cosmétique de traitement de la peau pour lutter contre les signes du vieillissement cutané, **caractérisé en ce qu'**il consiste à appliquer sur les zones de la peau nécessitant un tel traitement, une composition cosmétique topique comprenant une quantité efficace de l'association d'un extrait hydroglycériné de *Passiflora incarnata* et d'un extrait hydroglycériné d'*Anchusa arvensis,* lesdits extraits étant obtenus par percolation à partir des parties aériennes des plantes, et en combinaison le cas échéant avec des supports et excipients pharmaceutiquement et cosmétiquement acceptables.

## Patentansprüche

1. Kosmetische und/oder dermatologische Zusammensetzung, umfassend in Kombination einen Hydroglycerinextrakt von *Passifloraincarnata* und einen Hydroglycerinextrakt von *Anchusaarvensis,* wobei diese Extrakte durch Perkolation ab den oberirdischen Teilen der Pflanzen gewonnen werden, und gegebenenfalls in Kombination mit pharmazeutisch und kosmetisch akzeptablen Trägern und Hilfsstoffen.

2. Zusammensetzungnach Anspruch 1, **dadurch gekennzeichnet**, dassdas Gewichtsverhältnis des Hydroglycerinextrakts von *Passifloraincarnata* zum Hydroglycerinextrakt von *Anchusaarvensis* von 1 :30 bis 6: 1 schwankt.

3. Zusammensetzung nach Anspruch 2, **dadurch gekennzeichnet, dass** das Gewichtsverhältnis des Hydroglycerinextrakts von *Passifloraincarnata* zum Hydroglycerinextrakt von *Anchusaarvensis* von 1 :1 bis 4 : 1 schwankt.

4. Zusammensetzungnach Anspruch1, **dadurch gekennzeichnet**, dassdie Konzentration des Hydroglycerinextrakts von *Passifloraincarnata* in der Gesamtzusammensetzung von 0,1bis 3Gew.-% schwankt.

5. Zusammensetzung nach Anspruch 1, **dadurch gekennzeichnet, dass** die Konzentration des Hydroglycerinextrakts von *Anchusaarvensis* in der Gesamtzusammensetzung von 0,2 bis 3 Gew.-% schwankt.

6. Zusammensetzungnach Anspruch 1, **dadurch gekennzeichnet, dass** sie eine Kombination von Hydroglycerinextrakten von *Passifloraincarnata* und von *Anchusaarvensis* umfasst, deren Konzentration von 0,1 bis 20 Gew.-% Trockenextrakt in Bezug zum Gesamtgewicht der Zusammensetzung schwankt.

7. Verwendung eines Hydroglycerinextrakts von *Passifloraincarnata* in Kombination mit einem Hydroglycerinextrakt von *Anchusaarvensis,* wobei die Extrakte durch Perkolation ab den oberirdischen Teilen der Pflanzen für die Herstellung einer kosmetischen und/oder dermatologischen Zusammensetzung gewonnen werden, die zur Bekämpfung von Alterungserscheinungen der Haut bestimmt ist.

8. Verwendung nach Anspruch7, **dadurch gekennzeichnet**, dassdie Zusammensetzungzur Bekämpfung von Ausdrucksfalten bestimmt ist, die von den unkontrollierten Gesichtsmuskelkontraktionen hervorgerufen werden.

9. Kosmetisches Verfahren zur Behandlung der Haut zur Bekämpfung von Alterungserscheinungen der Haut, **dadurch gekennzeichnet, dass** es darin besteht, auf die Zonen der Haut, die eine derartige Behandlung benötigen, eine topische kosmetische Zusammensetzungaufzutragen, die eine wirksame Menge der Kombination eines Hydroglycerinextrakts von *Passifloraincarnata* und eines Hydroglycerinextrakts von *Anchusaarvensis* umfasst, wobei diese Extrakte durch Perkolation ab den oberirdischen Teilen der Pflanzen gewonnen werden, und gegebenenfalls in Kombination mit pharmazeutisch und kosmetisch akzeptablen Trägern und Hilfsstoffen.

## Claims

1. A cosmetic and/or dermatological composition comprising in association a hydroglycerin extract of *Passiflora incarnata* and a hydroglycerin extract of *Anchusa arvensis,* said extracts being obtained via percolation from the aerial parts of the plants, and optionally in combination with pharmaceutically and cosmetically acceptable excipients and carriers.

2. The composition according to claim 1, **characterized in that** the weight ratio of the hydroglycerin extract of *Passiflora incarnata* to the hydroglycerin extract of *Anchusa arvensis* varies from 1:30 to 6:1.

3. The composition according to claim 2, **characterized in that** the weight ratio of the hydroglycerin extract of *Passiflora incarnata* to the hydroglycerin extract of *Anchusa arvensis* varies from 1:1 to 4:1.

4. The composition according to claim 1, **characterized in that** the concentration of hydroglycerin extract of *Passiflora incarnata* in the total composition varies from 0.1 weight % to 3 weight %.

5. The composition according to claim 1, **characterized in that** the concentration of hydroglycerin extract of *Anchusa arvensis* in the total composition varies from 0.2 weight % to 3 weight %.

6. The composition according to claim 1, **characterized in that** it comprises an association of hydroglycerin extracts of *Passiflora incarnata* and *Anchusa arvenis* having a concentration varying from 0.1 to 20 weight % dry extract relative to the total weight of the composition.

7. The use of a hydroglycerin extract of *Passiflora incarnata* associated with a hydroglycerin extract of *Anchusa arvensis,* said extracts being obtained via percolation from the aerial parts of the plants to prepare a cosmetic and/or dermatological composition intended to combat signs of skin ageing.

8. The use according to claim 7, **characterized in that** the composition is intended to combat expression lines caused by uncontrolled facial muscle contractions.

9. A cosmetic skin treatment method to combat signs of skin ageing, **characterized in that** it consists of applying to skin areas requiring such treatment a topical cosmetic composition comprising an efficient amount of the association of a hydroglycerin extract of *Passiflora incarnata* and a hydroglycerin extract of *Anchusa arvenis,* said extracts being obtained via percolation from the aerial parts of the plants, optionally in combination with pharmaceutically and cosmetically acceptable excipients and carriers.
